# EUROPEAN PATENT APPLICATION

(11) **EP 4 667 937 A1**
(43) Date of publication of application: **24.12.2025**
(21) Application number: 25177513.6
(22) Date of filing: 20.05.2025
(51) Int. Cl.: G01N 33/53, G01N 33/543

(54) **TARGET MOLECULE DETECTION KIT AND TARGET MOLECULE DETECTION METHOD**

(30) Priority: 06.06.2024 JP 2024092467; 27.11.2024 JP 2024206657; 18.03.2025 JP 2025043964
(71) Applicant: DENSO CORPORATION, Kariya-city, Aichi 448-8661 (JP)
(72) Inventor: YOSHIMITSU, Yuuya, Kariya-city 448-8661 (JP); NAKAGAWA, Kazuhisa, Kariya-city 448-8661 (JP); NUKAZUKA, Akira, Kariya-city 448-8661 (JP); YOKOI, Takanori, Kariya-city 448-8661 (JP); NAKAMURA, Kazuya, Kariya-city 448-8661 (JP); HAYAKAWA, Kei, Kariya-city 448-8661 (JP)
(74) Representative: Kuhnen & Wacker Patent- und Rechtsanwaltsbüro PartG mbB

(57) **Abstract**

A target molecule detection kit used to detect a target molecule (10) includes a first binding element (20) and a second binding element (40). The first binding element is immobilized on a solid phase (30). The first binding element has reaction specificity to a first specific binding site of the target molecule. The second binding element is connected to a label (50) that generates a detection signal. The second binding element having reaction specificity to a second specific binding site of the target molecule that is different from the first specific binding site.

## Description

### TECHNICAL FIELD

The present disclosure relates to a target molecule detection kit and a target molecule detection method.

### BACKGROUND

Techniques to detect a target molecule, such as a specific antigen associated with a disease, in a sample have been known. For example, JP 2023-160814 A discloses an ELISA method (Enzyme Linked Immunosorbent Assay) as a method for capturing a target antigen contained in a sample solution with a specific antibody and detecting the target antigen using an enzyme reaction. Furthermore, Patent Document 1 discloses a technique to improve detection sensitivity and reactivity compared to the ELISA method. In this technique, a target substance is sandwiched between a first capture substance, which is connected to a label, and a second capture substance, which is immobilized on a solid phase, to form a complex. Then, the portion containing the label is separated from the formed complex, and the separated portion containing the label is moved through a liquid-filled tubular passage by centrifugal force. Thereafter, the portion containing the label is detected by scattered light irradiated into the tubular passage.

### SUMMARY

The technology disclosed in JP 2023-160814 A uses two types of capture substances: a first capture substance and a second capture substance. However, JP 2023-160814 A only discloses technical idea of using the first capture substance to fix the target substance and the second capture substance to detect the target substance. In other words, there is no technical idea of using these two types of capture substances to increase selectivity. Selectivity refers to the ability to bind appropriately to a desired target in a mixture. The same applies to the ELISA method. Thus, in the technique disclosed in Patent Document 1, the first capture substance and the second capture substance may bind to the same site on the target substance. Thus, the technique disclosed in JP 2023-160814 A may not be able to ensure high selectivity for the target substance.

One objective of the present disclosure is to provide a target molecule detection kit and a target molecule detection method that enable easier detection of a target molecule while ensuring higher selectivity for the target molecule.

The above objective is achieved by the combination of features described in the independent claims, and the dependent claims define further advantageous specific examples of the disclosure. Here, a reference numeral in parentheses in claims indicate a correspondence relationship with specific means described in embodiments to be described later as one aspect, and does not limit the technical scope of the present disclosure.

In order to achieve the above-mentioned objective, the target molecule detection kit used to detect a target molecule of the present disclosure includes a first binding element and a second binding element. The first binding element is immobilized on a solid phase and has reaction specificity to a first specific binding site of the target molecule. The second binding element is connected to a label that generates a detection signal and has reaction specificity to a second specific binding site of the target molecule. The second specific binding site is different from the first specific binding side.

According to the above configuration, since the first binding element is immobilized on the solid phase, the target molecule bound to the first binding element is easily extracted by capturing the target molecule with the first binding element. Furthermore, since the second binding element is connected to a label that generates a detection signal, the target molecule bound to the second binding element is easily detected by capturing the target molecule with the second binding element. Thus, the target molecule is more easily detected by binding both the first binding element and the second binding element to a target molecule. Furthermore, the first binding element and the second binding element each have reaction specificity to different binding sites on the same target molecule. Thus, high selectivity can be ensured by the reaction specificity to one of the binding sites even if the reaction specificity to the other of the binding sites is weak. Thus, it is possible to ensure higher selectivity for the target molecule, as compared with a configuration in which the first binding element and the second binding element have reaction specificity to the same binding site. As a result, the target molecule can be more easily detected while ensuring higher selectivity for the target molecule.

In order to achieve the above-mentioned objective, the target molecule detection method to detect a target molecule of the present disclosure includes reacting the target molecule having a first specific binding site and a second specific binding side with a first binding element immobilized on a solid phase and a second binding element connected to a label that generates a detection signal. The first binding element has reaction specificity to the first specific binding site. The second binding element has reaction specificity to the second specific binding site that is different from the first specific binding site.

According to the above configuration, since the first binding element is immobilized on the solid phase, the target molecule bound to the first binding element is easily extracted by capturing the target molecule with the first binding element. Furthermore, since the second binding element is connected to a label that generates a detection signal, the target molecule bound to the second binding element is easily detected by capturing the target molecule with the second binding element. Thus, the target molecule can be detected more easily by binding both the first binding element and the second binding element to the target molecule. Furthermore, the first binding element and the second binding element each have reaction specificity to different binding sites on the same target molecule. Thus, high selectivity can be ensured by the reaction specificity to one of the binding sites even if the reaction specificity to the other of the binding sites is weak. Thus, it is possible to ensure higher selectivity for the target molecule, as compared with a configuration in which the first binding element and the second binding element have reaction specificity to the same binding site. As a result, the target molecule can be more easily detected while ensuring higher selectivity for the target molecule.

### BRIEF DESCRIPTION OF THE DRAWINGS

FIG. 1 is a schematic diagram for explaining an example of a complex that captures and detects a target molecule.
FIG. 2 is a schematic diagram for explaining another example of a complex that captures and detects a target molecule.
FIG. 3 is a diagram for explaining a hydrolysis reaction in which ALP is used as a catalyst to generate hydrogen ions as a detection signal.
FIG. 4 is a diagram for explaining an example of a combination of a first binding element and a second binding element.
FIG. 5 is a diagram for explaining an example of a combination of a first binding element and second binding elements.
FIG. 6 is a diagram for explaining an example of a combination of a first binding element and a second binding element.
FIG. 7 is a diagram for explaining an example of a combination of a first binding element and second binding elements.
FIG. 8 is a flowchart showing an example of a protocol for a target molecule detection method.
FIG. 9 is a schematic diagram specifically illustrating each step of the target molecule detection method.
FIG. 10 is a flowchart showing an example of a detailed protocol of a reaction step in a first embodiment.
FIG. 11 is a graph for explaining the high selectivity of a target molecule by the target molecule detection method.
FIG. 12 is a graph for explaining the high detection sensitivity of the target molecule detection method.
FIG. 13 is a graph for explaining the high detection sensitivity of the target molecule detection method.
FIG. 14 is a graph for explaining the high selectivity of a target molecule by the target molecule detection method.
FIG. 15 is a graph for explaining the high detection sensitivity of the target molecule detection method.
FIG. 16 is a graph for explaining the high detection sensitivity of the target molecule detection method.
FIG. 17 is a graph for explaining the high detection sensitivity of the target molecule by the target molecule detection method.
FIG. 18 is a graph for explaining the high detection sensitivity of the target molecule detection method.
FIG. 19 is a flowchart showing an example of a detailed protocol of a reaction step in a second embodiment.

### DESCRIPTION OF EMBODIMENTS

With reference to the drawings, multiple embodiments for the disclosure will be described. For convenience of explanation, parts that have the same function as those shown in the figures used in the previous descriptions may be denoted by the same reference numerals, and their descriptions may be omitted. For parts denoted by the same reference numerals, the descriptions in other embodiments can be referred to.

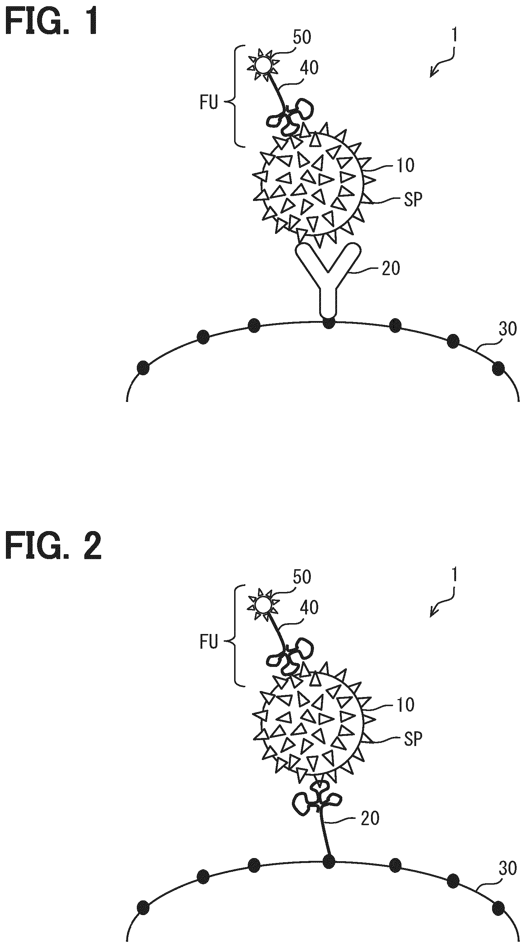

(First embodiment) In the target molecule detection method of the present disclosure, two or more types of binding elements for different binding sites are used to exclude molecules other than the target molecule, and to capture and detect specifically the target molecule with high sensitivity. The target molecule detection method of the present disclosure includes forming a sandwich complex 1 as shown in FIG. 1 to achieve above objectives. FIG. 1 is a schematic diagram illustrating an example of the sandwich complex 1 that captures and detects a target molecule 10. As shown in FIG. 1, the sandwich complex 1 includes the target molecule 10, a first binding element 20, a solid phase 30, a second binding element 40, and a label 50. As shown in FIG. 1, a fused body of the second binding element 40 and the label 50 is referred to as a label-fused body FU.

<Target Molecule 10> In the present disclosure, the target molecule 10 in this disclosure refers to a molecule to be detected in a sample. A sample refers to any solution, substance, or mixture that contains multiple molecules and may contain the target molecule 10. The target molecule 10 may be of various types. Examples of the target molecule 10 include viruses, cells, antigens, proteins, sugar chains, small molecules, bacteria, antibodies, lipids, peptides, and nucleic acids. The target molecule 10 may be a pathogen itself, such as a virus or bacterium, or may be a part of the pathogen, such as a protein or nucleic acid.

<First Binding Element 20> The first binding element 20 captures the target molecule 10 on the solid phase 30. The first binding element 20 is immobilized on the solid phase 30. The first binding element 20 has reaction specificity to a specific binding site (hereinafter, referred to as a first specific binding site) of the target molecule 10. The first binding element 20 immobilized on the solid phase 30 binds to the first specific binding site of the target molecule 10, so that the first binding element captures the target molecule 10 onto the solid phase 30.

The solid phase 30 is a member for facilitating separation of the target molecule 10 from non-target molecules other than the target molecule 10. The solid phase 30 may also be referred to as a fixing member. The solid phase 30 may be magnetic beads, resin beads, nanoparticles, plates, filters, semiconductors, sensitive membranes, metals, glasses, or ceramics. The plate may be a resin plate. The filter may be a paper filter or a fiber filter. The solid phase 30 allows the target molecule 10 captured on the solid phase 30 to be separated easily from non-target molecules. For example, magnetic beads allow separation of the target molecule 10 from non-target molecules by magnetic force. Resin beads or nanoparticles allow separation of the target molecule 10 from non-target molecules by centrifugation. Plates allow separation of the target molecule 10 from non-target molecules by washing. FIG. 1 shows an example in which the solid phase 30 is a magnetic bead. The explanation will be continued with the example in which the solid phase 30 is a magnetic bead. The magnetic beads can also be referred to as magnet beads. The first binding element 20 may be connected to the solid phase 30 by a covalent bond. As an example, when the first binding element 20 is an antibody and the solid phase 30 is a magnetic bead, the first binding element 20 may be immobilized on the solid phase 30 by covalent bonding between the amino group of the antibody and the carboxyl group of the magnetic bead.

The first binding element 20 may be an antibody or an aptamer. The antibody referred to here also includes antibody fragments and modified antibodies that have substantially the same reactivity as the antibody. The aptamer may be a nucleic acid aptamer or a peptide aptamer. The nucleic acid aptamer may be a DNA aptamer or an RNA aptamer. The nucleic acid aptamer may include an artificial nucleic acid. An aptamer is a nucleic acid molecule or peptide that specifically binds to a particular molecule. When an antibody is used as the first binding element 20 and a pathogen is used as the target molecule 10, the binding site of the target molecule 10 is, for example, an epitope. When an aptamer is used as the first binding element 20, the first binding site of the target molecule 10 is a region to which the aptamer can specifically bind, such as a receptor binding domain. FIG. 1 shows an example in which the first binding element 20 is an antibody and the target molecule 10 is a virus. In FIG. 1, SP denotes the spike protein of the virus which is the target molecule 10. FIG. 2 shows an example in which the first binding element 20 is an aptamer and the target molecule 10 is a virus. In FIG. 2, SP denotes the spike protein of the virus which is the target molecule 10.

The first binding element 20 having reaction specificity to the first binding site of the target molecule 10 may be generated as follows. For example, when the first binding element 20 is an antibody, the antibody may be generated by injecting an animal with an antigen containing the first specific binding site but not a second specific binding site, which will be described later. Alternatively, the antibody may be generated by phage display techniques. The antibody serving as the first specific binding element 20 may be a polyclonal antibody or a monoclonal antibody. From the viewpoint of reaction specificity, the antibody serving as the first specific binding element 20 is preferably a monoclonal antibody. When the first binding element 20 is a nucleic acid aptamer, the first binding element 20 having reaction specificity to the first specific binding site may be generated by the SELEX (Systematic Evolution of Ligands by EXponential enrichment) method. When the first binding element 20 is a peptide aptamer, the first binding element 20 having reaction specificity to the first specific binding site may be generated by polysome display or mRNA display.

<Second Binding Element 40> The second binding element 40 of the present disclosure is connected to the label 50 that generates a detection signal. The details of the label 50 will be described later. The second binding element 40 has reaction specificity to a binding site (hereinafter, referred to as a second specific binding site) different from the first specific binding site of the target molecule 10. The second binding element 40 connected to the label 50 binds to the second specific binding site of the target molecule 10, so that the target molecule 10 can be detected easily. The second binding element 40 may be connected to the label by a covalent bond.

The second binding element 40 may be an antibody or an aptamer, as described for the first binding element 20. The second binding element 40 having reaction specificity to the second specific binding site of the target molecule 10 may be generated in a similar manner to the generation of the first binding element 20 having reaction specificity to the first specific binding site described above, except that the target of the reaction specificity is different. FIG. 1 shows an example in which the second binding element 40 is an aptamer. FIG. 2 shows an example in which the second binding element 40 is an aptamer. The size ratio between the first binding element 20 and the second binding element 40 in FIG. 1 and the following figures is different from the actual ratio since antibodies and aptamers generally differ in size.

<Label 50> The label 50 is a substance that makes it easier to detect the target molecule 10. The label 50 generates a detection signal, thereby allowing easy detection of the target molecule 10 to which the second binding element 40, connected to the label 50, binds. The detection signal may be any signal that can be detected by existing equipment. The detection signal can also be referred to as a signal. The detection signals include ions, electric signals, heat, aggregation, fluorescence, and dyes. The label 50 may be a substance that generates a detection signal by itself, or may be an enzyme, DNAzyme, or RNAzyme that functions as a catalyst to generate a detection signal. FIG. 1 and 2 show an example in which the label 50 is an enzyme. Examples of the enzyme as the label 50 include peroxidase (HRP) and alkaline phosphatase (ALP). HRP catalyzes the chemical reaction of the substrate and produces a dye as a detection signal. ALP catalyzes the chemical reaction of the substrate and generates hydrogen ions as a detection signal.

The label 50 is preferably an enzyme that undergoes a chemical reaction with a substrate to produce ions as a detection signal. Using ions as a detection signal is easier to handle than using radiation as a detection signal. Furthermore, by using ions as a detection signal, it is possible to increase the detection sensitivity of the target molecule 10 compared to using fluorescence or dyes as a detection signal. For example, the target molecule 10 can be quantified with higher accuracy than when fluorescence or dyes are used as detection signals. In other words, it is possible to achieve more sensitive detection and more accurate quantification than the ELISA method, which estimates the concentration of a target protein based on changes in the absorption spectrum of a dye. More preferably, the label 50 is an enzyme that generates hydrogen ions as a detection signal by causing a hydrolysis reaction of a substrate to occur. In this case, the substrate is reacted with water. Thus, steps of preparing other substances than water for the chemical reaction of the substrate can be omitted, compared to cases other than hydrolysis reactions. Thus, the target molecule 10 can be detected more easily and accurately.

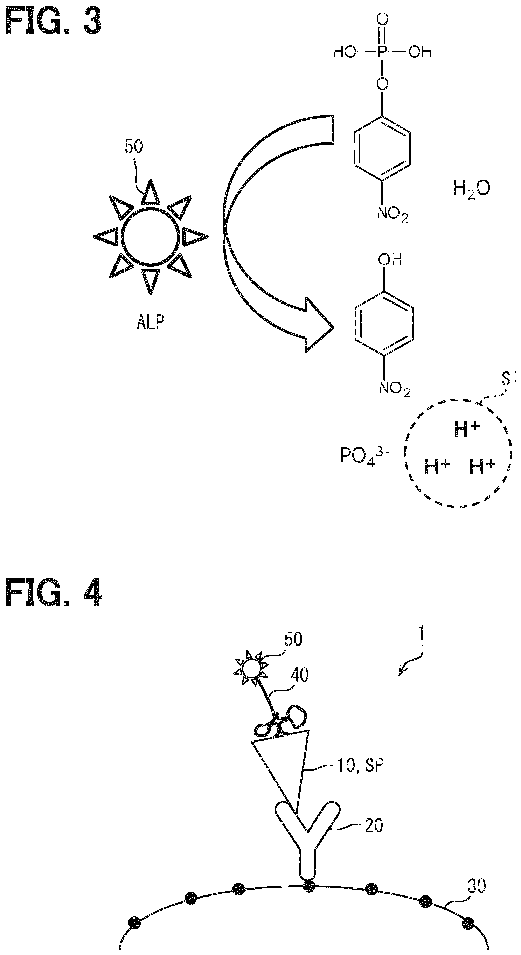

For example, the above-mentioned ALP may be used as an enzyme that generates hydrogen ions as a detection signal by causing a hydrolysis reaction of a substrate to occur. As the substrate, p-nitrophenyl phosphate may be used. Here, the hydrolysis reaction in the case where the label 50 is ALP and the substrate is p-nitrophenyl phosphate will be described with reference to FIG. 3. In FIG. 3, Si represents hydrogen ions generated in the hydrolysis reaction.

As shown in FIG. 3, when the substrate p-nitrophenyl phosphate are hydrolyzed with water using ALP which is the label 50 as a catalyst, p-nitrophenol, phosphate ions, and hydrogen ions are generated. As shown in FIG. 3, multiple hydrogen ions are generated from one p-nitrophenyl phosphate, and thus the detection signal is enhanced. Thus, the above configuration enhances the sensitivity of detection of the target molecule 10. In the following, the explanation will be continued with an example in which the label 50 is ALP and the detection signal is hydrogen ion. Ions other than hydrogen ions may be used as the detection signal as long as they are generated in a chemical reaction catalyzed by the label 50.

<Combination of First Binding Element 20 and Second Binding Element 40> The first binding element 20 and the second binding element 40 each have reaction specificity to different binding sites of the same target molecule 10. Thus, high selectivity can be ensured by the reaction specificity to one of the binding sites even if the reaction specificity to the other of the binding sites is weak. Thus, it is possible to ensure higher selectivity to the target molecule 10 compared to a configuration in which the first binding element 20 and the second binding element 40 have reaction specificity to the same binding site. The same applies to the case where both the first binding element 20 and the second binding element 40 are antibodies.

However, in order to ensure even higher selectivity for the target molecule 10, the followings are preferable. It is preferable that one of the first binding element 20 and the second binding element 40 is an aptamer, and the other is an aptamer or an antibody. In this manner, the first specific binding site and the second specific binding site can be completely different sites by making the first binding element 20 and the second binding element 40 different in type. Thus, it is easier to design that the first binding element 20 and the second binding element 40 to bind to completely different binding sites. As a result, it is possible to ensure even higher selectivity for the target molecule 10. In addition, it is difficult to prepare two types of antibodies against one target molecule. Thus, it is preferable that either the first binding element 20 or the second binding element 40 be an aptamer.

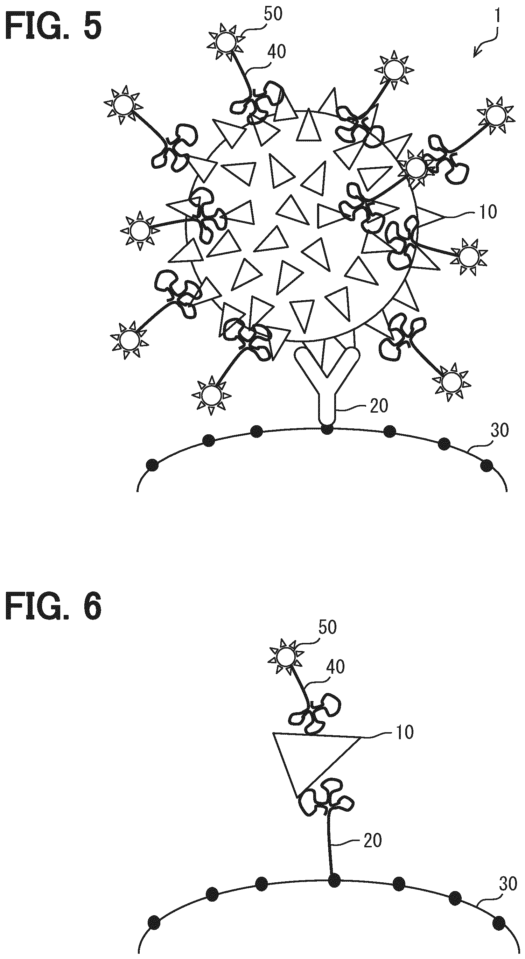

Here, an example of a combination of the first binding element 20 and the second binding element 40 will be described with reference to FIGS. 4 to 7. For example, as shown in FIGS. 4 and 5, the first binding element 20 may be an antibody, and the second binding element 40 may be an aptamer. FIG. 4 shows an example in which the target molecule 10 is a part of a pathogen and is separated from the pathogen. In the example of FIG. 4, the target molecule 10 is the spike protein SP of a virus. In the example of FIG. 4, the spike protein SP has an epitope as the first specific binding site, and the antibody as the first binding element 20 binds to the epitope as the first specific binding site. On the other hand, the spike protein SP has a second specific binding site, and an aptamer as the second binding element 40 binds to the second specific binding site of the spike protein SP. Due to these connections, the spike protein SP is sandwiched between the first binding element 20 and the second binding element 40. The first binding element 20 is an antibody and the second binding element 40 is an aptamer, so that higher selectivity for the target molecule 10 can be ensured, as described above.

FIG. 5 shows an example in which the target molecule 10 is part of a pathogen and remains contained within the pathogen. In the example of FIG. 5, the target molecule 10 is the spike protein SP of a virus. In the example of FIG. 5, an antibody as the first binding element 20 binds to the first binding site, which is the epitope of the spike protein SP. On the other hand, the spike protein SP has a second specific binding site, and an aptamer as the second binding element 40 binds to the second specific binding site. In addition, the virus has multiple spike proteins SP. Since the second binding element 40 is an aptamer which is smaller in size than an antibody, multiple second binding elements 40 binds to one virus as shown in FIG. 5. The multiple second binding elements 40, which are aptamers, are each connected to the label 50, and the multiple second binding elements 40 bind to a pathogen, thereby improving the detection sensitivity of the pathogen.

FIG. 6 shows an example in which the target molecule 10 is a part of a pathogen and is separated from the pathogen. In the example of FIG. 6, the target molecule 10 is the spike protein SP of a virus. In the example of FIG. 6, the aptamer as the first binding element 20 binds to the epitope of the spike protein SP as the first binding site. On the other hand, the spike protein SP has a second specific binding site, and an aptamer as the second binding element 40 binds to the second specific binding site. Due to these connections, the spike protein SP is sandwiched between the first binding element 20 and the second binding element 40. Even when the first binding element 20 is an aptamer and the second binding element 40 is an aptamer, high selectivity for the target molecule 10 can be ensured compared to when both are antibodies.

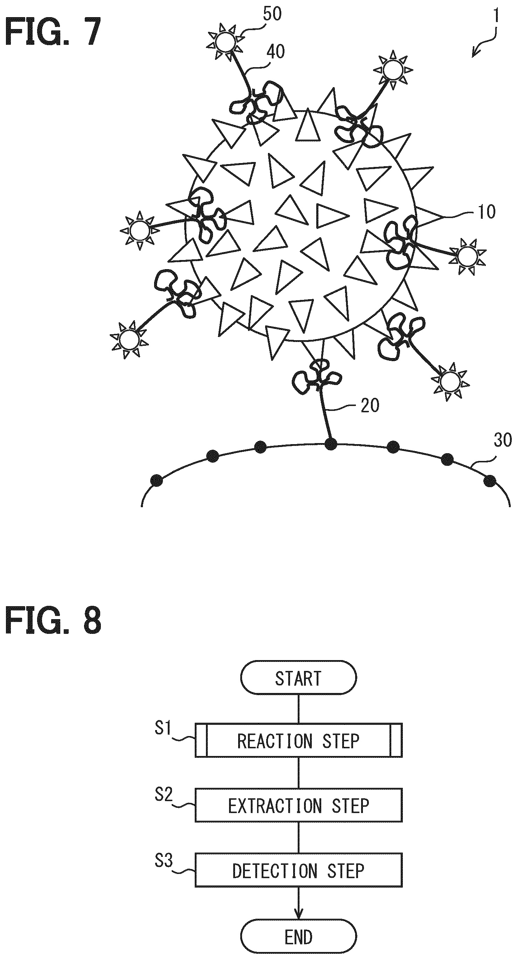

Although an example in which an antibody is used as the first binding element 20 is shown in FIGS. 4 and 5, the first binding element 20 may be an aptamer, as described above. For example, as shown in FIG. 7, both the first binding element 20 and the second binding element 40 may be aptamers. FIG. 7 shows an example in which the target molecule 10 is a part of a pathogen and remains contained within the pathogen. In the example of FIG. 7, the target molecule 10 is the spike protein of a virus. In the example of FIG. 7, the aptamer as the first binding element 20 binds to the first specific binding side of the spike protein SP. Additionally, the spike protein SP has a second specific binding site, and an aptamer as the second binding element 40 binds to the second specific binding site. In addition, the virus has multiple spike proteins SP. Thus, as shown in FIG. 7 as well as in FIG. 5, the multiple aptamers as the second binding element 40 bind to one virus.

When both the first binding element 20 and the second binding element 40 are aptamers, the followings are preferable. For example, it is preferable to use aptamers of different types, such as a combination of a nucleic acid aptamer and a peptide aptamer, or a combination of a DNA aptamer and an RNA aptamer. This makes it easier for the first specific binding site and the second specific binding site to be completely different binding sites. As a result, it is possible to ensure even higher selectivity for the target molecule 10. Furthermore, when both the first binding element 20 and the second binding element 40 are aptamers, the aptamers may be of the same type as long as the aptamers have different sequences.

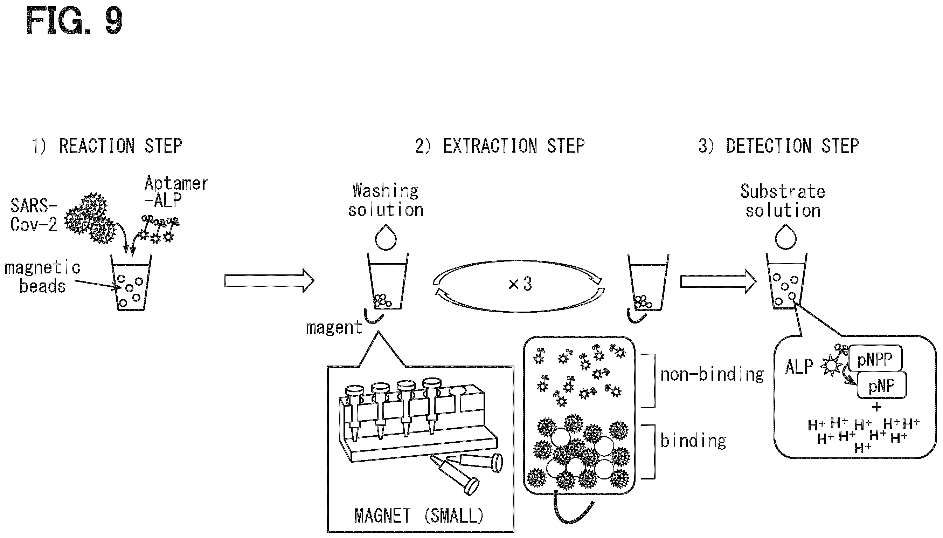

<Protocol> Next, an example of a protocol for the target molecule detection method of the present disclosure will be described with reference to FIGS. 8 to 10. FIG. 8 is a flowchart showing an example of a protocol for a target molecule detection method. FIG. 9 is a schematic diagram for specifically illustrating each step of the target molecule detection method. In FIG. 9, SARS-CoV2 virus is used as the target molecule 10 as an example. Here, the description will be given with a case where the first binding element 20 immobilized on the solid phase 30 and the second binding element connected to the label 50 are prepared in advance. For example, the first binding element 20 immobilized on the solid phase 30 and the second binding element connected to the label 50 may each be stored in a storage buffer. Since the composition of various buffers and temperature conditions vary depending on the types of the target molecule 10, the first binding element 20, and the second binding element 40, detailed explanations of these conditions are omitted. The following describes the procedure.

First, in step S1, a reaction step is carried out, and then the process proceeds to step S2. In the reaction step, the target molecule 10 is reacted with the first binding element 20 immobilized on the solid phase 30 and the second binding element 40 connected to the label 50. In the reaction step S1, as shown in FIG. 9, the target molecule 10 and the second binding element 40 connected to the label 50 is added to a reaction field including the first binding element 20 immobilized on a magnetic bead as the solid phase 30, and the above-mentioned reaction is carried out. The reaction field represents a partitioned space. The reaction field may be a reaction vessel or a reaction chamber. As an example, the reaction field may be a microtube. FIG. 9 shows an example in which a microtube is used as the reaction field.

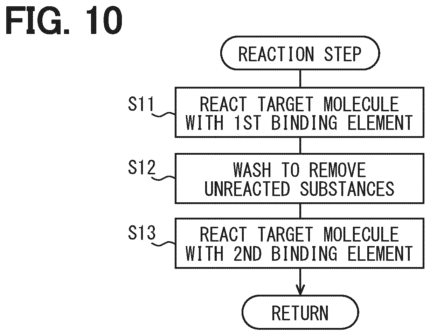

An example of a detailed protocol of the reaction step in the first embodiment will be described using the flowchart of FIG. 10. FIG. 10 is a flowchart showing an example of a detailed protocol of the reaction step in the first embodiment.

First, in step S11, a sample containing the target molecule 10 is added to a reaction field including the first binding element 20 immobilized on magnetic beads as the solid phase 30 to react the target molecule 10 with the first binding element 20.

In step S12, a washing solution is added to the reaction filed after the reaction in S11 for washing. The washing liquid may be a washing buffer. In S12, the complex formed of the first binding element 20 immobilized on the magnetic beads and the target molecule 10 binding to the first binding element 20 is captured by separating the complex from non-reacted substances by magnetic force of magnets. In S12, washing is performed to remove the non-reacted substances by adding a washing buffer to the reaction field and removing the supernatant. The supernatant may be removed by adding a washing buffer to the reaction field, stirring the mixture, and then collecting the precipitate by magnetic force. In S12, the process of adding a washing buffer to the reaction field and removing the supernatant may be repeated multiple times.

In step S13, the second binding element 40 connected to the label 50 is added to the reaction field after washing in S12, and the target molecule 10 and the second binding element 40 are reacted with each other. Then, the process proceeds to step S2. The complex formed of the first binding element 20 immobilized on the magnetic beads and the target molecule 10 binding to the first binding element 20 remains in the reaction field after the washing in S12 by the magnetic force of the magnet. The reaction in S13 forms the sandwich complex 1 that is formed of the complex in step S12 and the label-fused body FU. The label-fused body FU is formed of the label 50 and the second binding element 40 binding to the target molecule 10 of the complex. Thus, in the reaction step in the first embodiment, the target molecule 10 is first reacted with the first binding element 20, and then the target molecule 10 that has reacted with the first binding element 20 is reacted with the second binding element 40. More specifically, the target molecule 10 is first reacted with the first binding element 20, and then the first binding element 20 that has not bound to the target molecule 10 is removed. Then, the target molecule 10 that has bound to the first binding element 20 is reacted with the second binding element 40.

Between the steps of S12 and S13, a blocking process may be performed in which a blocking agent such as BSA (bovine serum albumin) is added to the reaction field. When blocking process has been performed, washing may be performed with a washing buffer to remove unreacted blocking agent from the reaction field. In the target molecule detection method of the present disclosure, the blocking process may not be performed. This is because the first binding element 20 and the second binding element 40 have reaction specificities to different specific binding sites of the target molecule 10, and the selectivity for the target molecule 10 is high even without performing a blocking process.

In addition, the flowchart in FIG. 10 shows a configuration in which the process S11, in which the target molecule 10 is reacted with the first binding element 20, is followed by the process S13, in which the target molecule 10 is reacted with the second binding element 40. However, the present disclosure is not necessarily limited to this. For example, the process of reacting the target molecule 10 with the second binding element 40 may be followed by the process of reacting the target molecule 10 with the first binding element 20. In other words, the target molecule 10 may be reacted with the second binding element 40 first, and the second binding element 40 that has not bound to the target molecule 10 may be removed, and then the target molecule 10 that has bound to the second binding element 40 may be reacted with the first binding element 20.

Returning to FIG. 8, in the extraction step S2, the sandwich complex 1 formed of the first binding element 10, the second binding element 40, and the target molecule 10 that has bound to both the first binding element 20 and the second binding element 40 in the reaction step S1 is extracted. In S2, a washing solution is added to the reaction filed after the reaction in S1 for washing. As described above, the washing liquid may be a washing buffer. In S2, as shown in FIG. 9, the sandwich complex 1 is separated from non-reacted substances and collected by the magnetic force of a magnet. In S2, as shown in FIG. 9, washing is performed to remove the non-reacted substances by adding a washing buffer to the reaction field and removing the supernatant. As shown in FIG. 9, the non-reacted substances are substances other than the sandwich complex 1 that is bound to the first binding element 20 immobilized on the magnetic beads. In S2, the process of adding a washing buffer to the reaction field and removing the supernatant may be repeated multiple times. For example, in the example of FIG. 9, the washing is repeated three times. The supernatant may be removed by adding a washing buffer to the reaction field, stirring the mixture, and then collecting the precipitate by magnetic force.

In the detection step of step S3, the target molecule 10 is detected using the label 50 connected to the second binding element 40 in the sandwich complex 1 that is extracted in the extraction step S2. In S3, the sandwich complex 1 remaining in the reaction field is reacted with the substrate solution by the magnetic force of the magnet. The substrate solution may be a solution containing p-nitrophenyl phosphate and water, as shown in the example of FIG. 9. In the present embodiment, since the label 50 is ALP as shown in FIG. 9, hydrogen ions are generated by the above-mentioned hydrolysis reaction. In S3, the concentration of the hydrogen ions is measured by a hydrogen ion sensor to detect the target molecule 10. The hydrogen ion sensor may be a semiconductor sensor that detects hydrogen ions by utilizing a metal oxide semiconductor. The hydrogen ion sensor may be a pH sensor. In the detection step S3, the presence of the target molecule 10 may be detected, or the amount of the target molecule 10 may be quantified based on the concentration of hydrogen ions.

By applying the target molecule detection method to a sample in which it is uncertain whether the target molecule 10 is present or not, it becomes possible to determine the presence or absence of the target molecule 10 in the sample. For example, when the target molecule 10 is a pathogen or a part of a pathogen, it is possible to determine the presence or absence of the pathogen in a sample. This makes it suitable for use in testing for the presence or absence of pathogen infection in the human body.

<Target Molecule Detection Kit> The target molecule detection kit is used to detect the target molecule 10 by the target molecule detection method of the present disclosure. The target molecule detection kit may include the first binding element 20 immobilized on the solid phase 30 and the second binding element 40 connected to the label 50. According to this, the first binding element 20 is immobilized on the solid phase 30. Thus, the target molecule 10 binding to the first binding element 20 can be extracted more easily by capturing the target molecule 10 with the first binding element 20. Furthermore, since the second binding element 40 is connected to the label 50 that generates a detection signal, the target molecule 10 bound to the second binding element 40 is easily detected by capturing the target molecule 10 with the second binding element 40. Thus, binding of the target molecule 10 to both the first binding element 20 and the second binding element 40 can detect the target molecule 10 more easily. Furthermore, since the first binding element 20 and the second binding element 40 each have reaction specificity to different binding sites of the same target molecule 10, as described above, it is possible to ensure higher selectivity for the target molecule 10. As a result, the target molecule 10 can be detected more easily while ensuring higher selectivity for the target molecule 10.

The target molecule detection kit may contain a substrate for use in the detection. The target molecule detection kit may contain a washing solution for use in the above-mentioned washing. The target molecule detection kit may include a sensor that detects the target molecule 10 based on a detection signal generated by the label 50. The target molecule detection kit may include a hydrogen ion sensor.

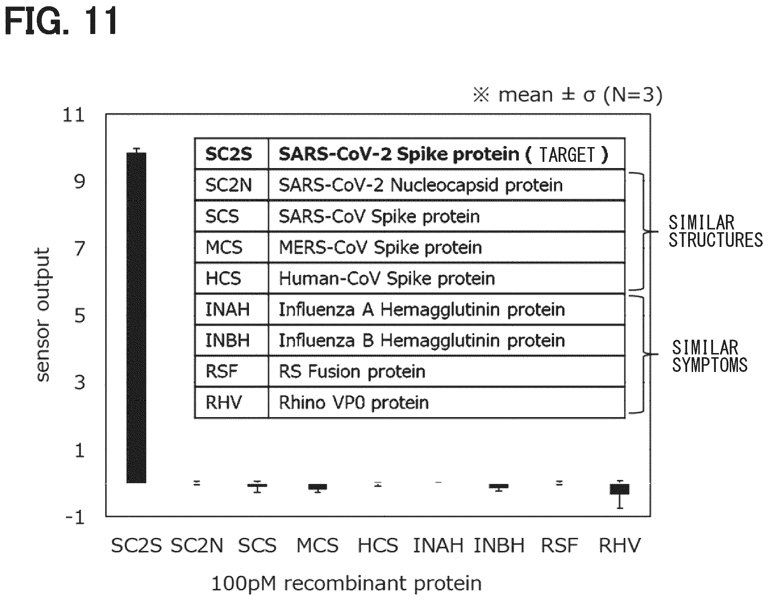

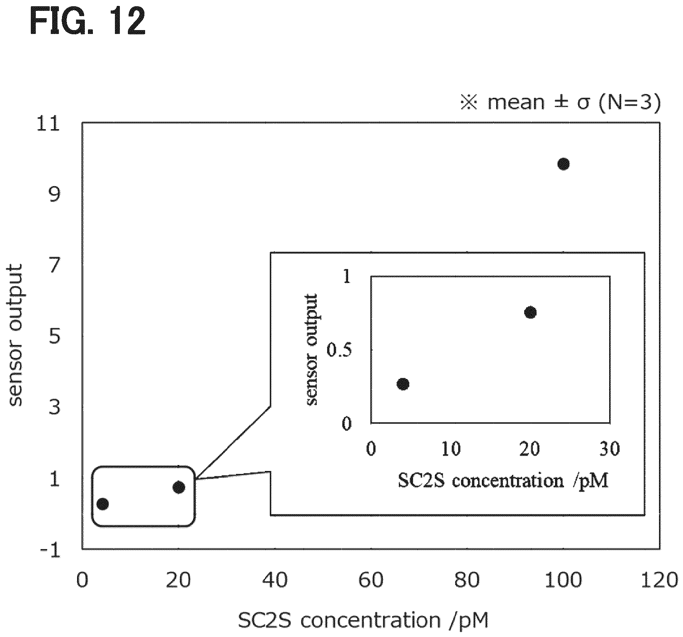

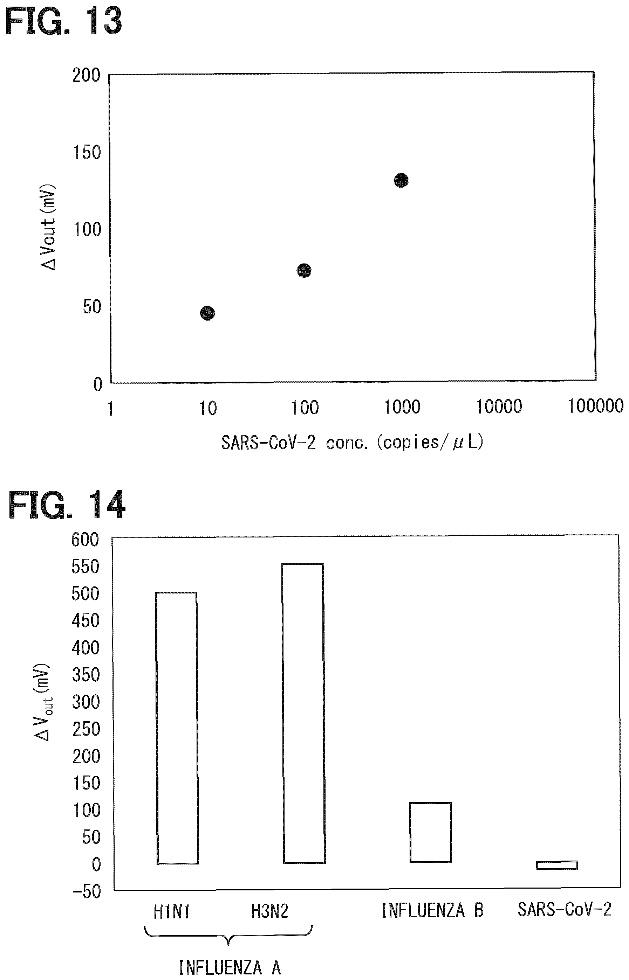

<Overview of First Embodiment> According to the configuration of the first embodiment, as described above, the target molecule 10 can be detected more easily while higher selectivity for the target molecule 10 is ensured. Here, the effect of the target molecule detection method of the present disclosure will be further explained with reference to FIGS. 11 to 18. FIG. 11 to 13 and 18 show examples in which an antibody is used for the first binding element 20, and a DNA aptamer is used for the second binding element 40. In FIG. 11 and 12, the spike protein of SARS-CoV-2 (hereinafter, SC2S) was used as the target molecule 10. In FIG. 13, SARS-CoV-2 itself was used as the target molecule 10. SARS-CoV-2 is a coronavirus that belongs to the SARS-associated coronavirus (SARSr-CoV) family. In the examples of FIGS. 11 to 18, ALP was used as the label 50, and p-nitrophenyl phosphate was used as the substrate. FIGS. 11 to 18 show examples in which hydrogen ions generated as a detection signal are detected by a hydrogen ion sensor.

FIG. 11 is a graph illustrating the high selectivity of the target molecule 10 using the target molecule detection method of the present disclosure. The vertical axis in FIG. 11 represents the detection value of the hydrogen ion sensor. The horizontal axis in FIG. 11 indicates the protein names of the samples. In FIG. 11, various types of samples other than the target SC2S are used as controls. The control samples were samples with a structure similar to SC2S and samples from viruses causing symptoms similar to those of SARS-CoV-2. As samples with similar structures, SC2N, SCS, MCS, and HCS were used. SC2N is the abbreviation for the nucleocapsid protein of SARS-CoV-2. SCS is the abbreviation for the spike protein of SARS-CoV. MCS is the abbreviation for the spike protein of MERS-CoV. HCS is the abbreviation for the spike protein of Human-CoV. As symptom-similar samples, INAH, INBH, RSF, and RHV were used. INAH is the abbreviation for influenza A hemagglutinin protein. INBH is the abbreviation for influenza B hemagglutinin protein. RSF is the abbreviation for the fusion protein of RS virus. RHV is the abbreviation for the VP0 protein of rhinovirus. In FIG. 11, detection is performed with the protein concentration of each sample set to 100 pM.

As shown in FIG. 11, with the target molecule detection method of the present disclosure, SC2S, which is the target molecule 10, was detected while other samples with a similar structure to SC2S or samples with similar symptoms were not detected. In other words, high selectivity was demonstrated in detecting the target molecule 10.

FIG. 12 is a graph illustrating the high detection sensitivity of the target molecule 10 by the target molecule detection method of the present disclosure. The vertical axis in FIG. 12 represents the detection value of the hydrogen ion sensor. The horizontal axis in FIG. 12 represents the concentration of SC2S. The concentration unit is pM. As shown in FIG. 12, according to the target molecule detection method of the present disclosure, a detection value of the hydrogen ion sensor having a magnitude corresponding to the concentration of the target molecules 10 is obtained. Thus, by determining in advance the correspondence relationship between the concentration of the target molecules 10 and the sensor detection value, the target molecule 10 can be quantified with high accuracy using this correspondence relationship. As shown in FIG. 12, this correspondence relationship is maintained even when the target molecule 10 is at a low concentration of several pM. Thus, as shown in FIG. 12, the target molecule detection method of the present disclosure makes it possible to detect the target molecule 10 with high sensitivity.

FIG. 13 is also a graph illustrating the high detection sensitivity of the target molecule 10 by the target molecule detection method of the present disclosure. The vertical axis in FIG. 13 represents the detection value of the hydrogen ion sensor. The horizontal axis of FIG. 13 represents the concentration of SARS-CoV-2. The unit of concentration is copies/µL. As shown in FIG. 13, according to the target molecule detection method of the present disclosure, even if the target molecule 10 is a virus itself, a detection value of the hydrogen ion sensor having a magnitude corresponding to the concentration of the target molecule 10 can be obtained. Thus, as shown in FIG. 13, according to the target molecule detection method of the present disclosure, the target molecule 10 can be detected with high sensitivity even if the target molecule 10 is not a protein extracted from a virus, but the virus itself.

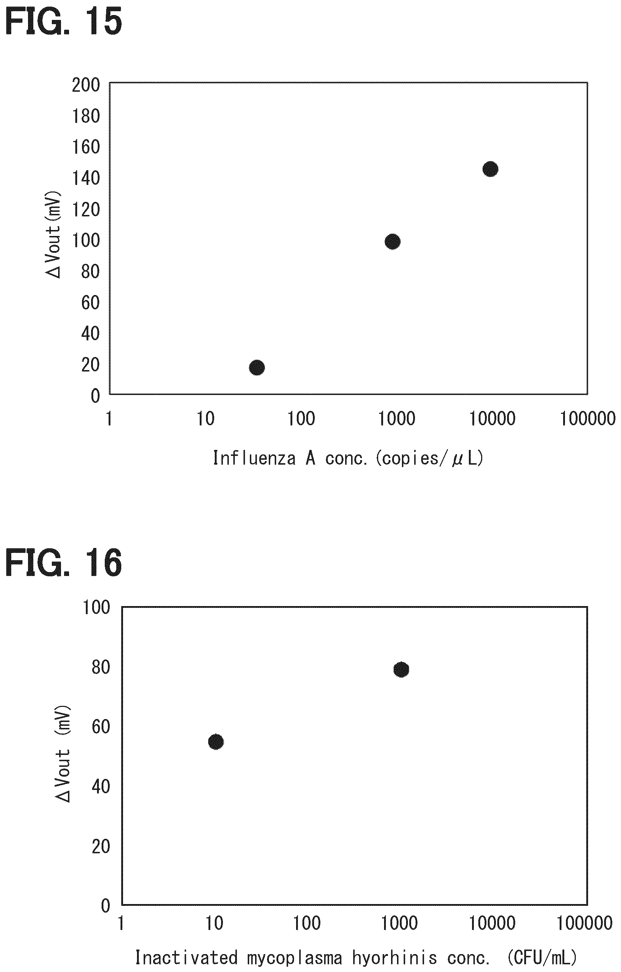

FIGS. 14 and 15 show an example in which both the first binding element 20 and the second binding element 40 are DNA aptamers. In FIGS. 14 and 15, the influenza A virus itself was used as the target molecule 10. Influenza A virus has two subtypes, H1N1 and H3N2. FIG. 14 and 15 show an example in which influenza A virus H1N1 is the detection target. In the examples of FIGS. 14 and 15, ALP was used as the label 50 and p-nitrophenyl phosphate was used as the substrate. FIG. 14 and 15 show an example in which hydrogen ions generated as a detection signal are detected by a hydrogen ion sensor.

FIG. 14 is a graph illustrating the high selectivity of the target molecule 10 by the target molecule detection method of the present disclosure. The vertical axis in FIG. 14 represents the detection value of the hydrogen ion sensor. The horizontal axis in FIG. 14 indicates the sample name. In FIG. 14, multiple types of samples other than the target influenza A virus H1N1 and H3N2 are used as controls. Influenza B virus itself and SARS-CoV-2 itself were used as the control samples.

As shown in FIG. 14, the target molecule detection method of the present disclosure detected the target influenza A virus H1N1 with high sensitivity. The same influenza A virus H2N3 is also detected with high sensitivity. On the other hand, influenza B virus, which is structurally similar but a different species from influenza A virus, was hardly detected. In addition, the detection limit for SARS-CoV-2, which is a very different species from influenza A virus, was below the limit of measurement. In other words, even when aptamers were used in both the first binding element 20 and the second binding element 40, high selectivity was exhibited in detecting the target molecule 10.

FIG. 15 is a graph illustrating the high detection sensitivity of the target molecule 10 by the target molecule detection method of the present disclosure. The vertical axis in FIG. 15 represents the detection value of the hydrogen ion sensor. The horizontal axis of FIG. 15 represents the concentration of H1N1. The unit of concentration is copies/µL. As shown in FIG. 15, according to the target molecule detection method of the present disclosure, a detection value of the hydrogen ion sensor having a magnitude corresponding to the concentration of the target molecules 10 is obtained. Thus, as shown in FIG. 14, according to the target molecule detection method of the present disclosure, the target molecule 10 can be detected with high sensitivity even when aptamers are used in both the first binding element 20 and the second binding element 40.

FIGS. 16 to 18 are graphs illustrating the high detection sensitivity of the target molecule 10 by the target molecule detection method of the present disclosure. FIG. 16 shows an example in which inactivated Mycoplasma hyorhinis is used as the target molecule 10. Inactivated mycoplasma is a type of mycoplasma, which is bacterium, that has been inactivated. FIG. 16 shows an example in which both the first binding element 20 and the second binding element 40 are DNA aptamers. The vertical axis in FIG. 16 represents the detection value of the hydrogen ion sensor. The horizontal axis of FIG. 16 represents the concentration of inactivated mycoplasma hyorhinis. The unit of concentration is CFU/mL. As shown in FIG. 16, according to the target molecule detection method of the present disclosure, a detection value of the hydrogen ion sensor having a magnitude corresponding to the concentration of the target molecules 10 is obtained. Thus, as shown in FIG. 16, according to the target molecule detection method of the present disclosure, the target molecule 10 can be detected with high sensitivity even when both the first binding element 20 and the second binding element 40 are aptamers and even when the target molecule 10 is an inactivated bacterium.

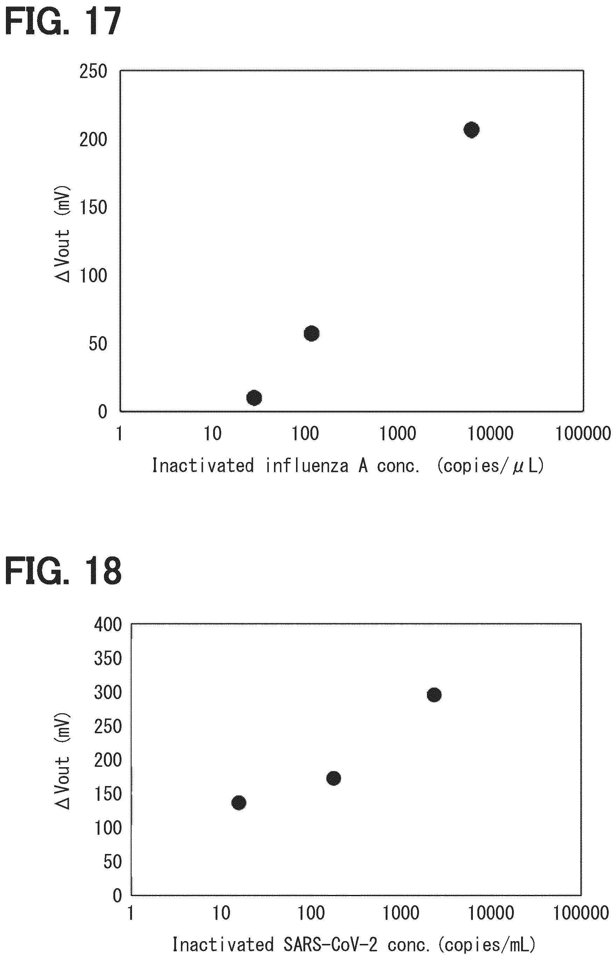

FIG. 17 shows an example in which inactivated influenza A is used as the target molecule 10. Inactivated influenza A is an inactivated influenza A virus. FIG. 17 shows an example in which both the first binding element 20 and the second binding element 40 are DNA aptamers. The vertical axis in FIG. 17 represents the detection value of the hydrogen ion sensor. The horizontal axis in FIG. 17 represents the concentration of inactivated influenza A. The unit of concentration is copies/µL. As shown in FIG. 17, according to the target molecule detection method of the present disclosure, a detection value of the hydrogen ion sensor having a magnitude corresponding to the concentration of the target molecules 10 is obtained. Thus, as shown in FIG. 17, according to the target molecule detection method of the present disclosure, the target molecule 10 can be detected with high sensitivity even when both the first binding element 20 and the second binding element 40 are aptamers and the target molecule 10 is an inactivated virus.

FIG. 18 shows an example in which inactivated SARS-CoV-2 is used as the target molecule 10. Inactivated SARS-CoV-2 is the virus SARS-CoV-2 that has been inactivated. FIG. 18 shows an example in which an antibody is used for the first binding element 20 while a DNA aptamer is used for the second binding element 40, as described above. The vertical axis in FIG. 18 represents the detection value of the hydrogen ion sensor. The horizontal axis in FIG. 18 represents the concentration of inactivated influenza A. The unit of concentration is copies/µL. As shown in FIG. 18, according to the target molecule detection method of the present disclosure, a detection value of the hydrogen ion sensor having a magnitude corresponding to the concentration of the target molecules 10 is obtained. Thus, as shown in FIG. 18, according to the target molecule detection method of the present disclosure, the target molecule 10 can be detected with high sensitivity even when the first binding element 20 is an antibody and the second binding element 40 is an aptamer, and even when the target molecule 10 is an inactivated virus.

Furthermore, the target molecule detection method of the present disclosure has the following advantages over ELISA and methods similar to ELISA. In the ELISA method, it is necessary to destroy a pathogen and extract a detection target, such as a protein, before carrying out detection. On the other hand, in the target molecule detection method of the present disclosure, it is possible to devise combinations of the first binding element 20 and the second binding element 40 to detect various detection targets. Thus, in the target molecule detection method of the present disclosure, it is possible to detect proteins and the like on the surface of pathogens as detection targets without destroying the pathogens themselves.

(Second Embodiment) In the first embodiment, the target molecule 10 is reacted with the first binding element 20 and the second binding element 40 in that order in the reaction step, but the present disclosure is not necessarily limited to this. The configuration of the following second embodiment may be applied. The followings will describe an example of the configuration of the second embodiment with reference to the accompanying drawings. The target molecule detection method of the second embodiment is similar to the target molecule detection method of the first embodiment, except that a part of the reaction process is different. Hereinafter, the different point will be described.

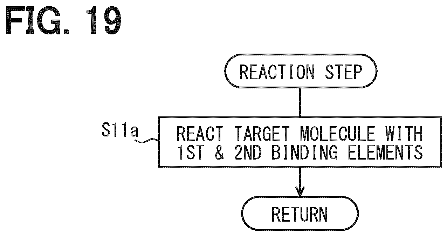

With reference to the flowchart of FIG. 19, an example of a detailed protocol of the reaction step in the second embodiment will be described. FIG. 19 is a flowchart showing an example of the detailed protocol of the reaction step in the second embodiment.

In step S11a, the second binging element 40 connected to the label 50 and a sample containing the target molecule 10 are added to a reaction field including the first binding element 20 immobilized on magnetic beads as the solid phase 30. In S11a, the first binding element 20 immobilized on the solid phase 30 and the second binding element 40 connected to the label 50 may be added to a reaction field including a sample containing the target molecule 10. That is, in S11a, the reaction between the target molecule 10 and the first binding element 20 and the reaction between the target molecule 10 and the second binding element 40 are caused to occur at the same timing. The same timing referred to here means approximately the same timing, and does not mean that the first binding element 20 and the second binding element 40 are added to the reaction field at completely the same timing. Either the first binding element 20 or the second binding element 40 may be added to the reaction field first. The same timing referred to here also includes a situation in which one of the first binding element 20 and the second binding element 40 is added to the reaction field and the other of the first binding element 20 and the second binding element 40 is added to the reaction field without removing unreacted binding element that was initially added.

In addition, in S11a, a blocking treatment in which a blocking agent such as BSA is added to the reaction field may be performed at the same timing. The blocking process may not be performed. This is because the first binding element 20 and the second binding element 40 have reaction specificities to different specific binding sites of the target molecule 10, and the selectivity for the target molecule 10 is high even without performing a blocking process.

Similarly to the first embodiment, the second embodiment also makes it possible to detect the target molecule 10 more easily while ensuring higher selectivity for the target molecule 10. Furthermore, according to the second embodiment, the reaction between the target molecule 10 and the first binding element 20 and the reaction between the target molecule 10 and the second binding element 40 are carried out at the same timing, making the procedure simpler. According to the target molecule detection method of the present disclosure, as shown in the first and second embodiments, the order in which the first binding element 20 and the second binding element 40 are mixed with the target molecule 10 can be freely changed. In this respect, the method has an advantage over ELISA and methods similar to ELISA.

(Third Embodiment) In the above embodiments, two types of binding elements, which are the first binding element 20 and the second binding element 40, having reaction specificity to different binding sites are explained. However, the present disclosure is not limited to this. For example, three or more types of binding elements having reaction specificity to different binding sites may be used. As an example, two or more types of binding elements having reaction specificity to different binding sites of the target molecule 10 may be used as the binding elements connected to the label 50. According to the above configuration, the probability that high selectivity can be ensured due to the reaction specificity of any one of the binding sites is increased. Thus, it is possible to ensure even higher selectivity for the target molecule 10.

The present specification discloses multiple technical concepts described in the multiple claims listed below. Some claims are described in a multiple dependent form, which optionally refers to preceding claims in subsequent claims. Furthermore, some claims are described in a multiple dependent form referring to another multiple dependent form. These claims described in such multiple dependent forms define multiple technical concepts.

(Technical concept 1) A target molecule detection kit used to detect a target molecule (10) is provided. The target molecule detection kit includes a first binding element (20) immobilized on a solid phase (30), and a second binding element (40) connected to a label (50) that generates a detection signal. The first binding element has reaction specificity to a first specific binding site of the target molecule. The second binding element has reaction specificity to a second specific binding site of the target molecule that is different from the first specific binding site.

(Technical concept 2) In the target molecule detection kit according to technical concept 1, one of the first binding element and the second binding element is an aptamer and the other of the first binding element and the second binding element is an aptamer or an antibody.

(Technical concept 3) In the target molecule detection kit according to technical concept 1 or 2, the label connected to the second binding element is an enzyme that generates ions as the detection signal by causing a chemical reaction of a substrate.

(Technical concept 4) In the target molecule detection kit according to technical concept 3, the enzyme, as the label connected to the second binding element, generates hydrogen ions by causing a hydrolysis reaction of the substrate.

(Technical concept 5) A target molecule detection method to detect a target molecule (10) is provided. The target molecule detection method includes reacting the target molecule with a first binding element (20) immobilized on a solid phase, and a second binding element (40) connected to a label (50) that generates a detection signal. The first binding element has reaction specificity to a first specific binding site of the target molecule, and the second binding element has reaction specificity to a second specific binding site of the target molecule that is different from the first specific binding site.

(Technical concept 6) The target molecule detection method according to technical concept further includes extracting a complex (1) of the target molecule having bound to both the first binding element and the second binding element, and detecting the target molecule using the label connected to the second binding element included in the extracted complex.

(Technical concept 7) In the target molecule detection method according to technical concept 6, the reacting of the target molecule with the first binding element and the second binding element includes reacting the target molecule with the first binding element, and then reacting the target molecule with the second binding element, or reacting the target molecule with the second binding element, and then reacting the target molecule with the first binding element.

(Technical concept 8) In the target molecule detection method according to technical concept 6, the reacting of the target molecule with the first binding element and the second binding element includes causing a reaction between the target molecule and the first binding element and a reaction between the target molecule and the second binding element to occur at a same timing.

(Technical concept 9) In the target molecule detection method according to any one of technical concepts 5 to 8, one of the first binding element and the second binding element is an aptamer and the other of the first binding element and the second binding element is an aptamer or an antibody.

(Technical concept 10) In the target molecule detection method according to any one of technical concepts 5 to 8, the label connected to the second binding element is an enzyme that generates ions as the detection signal by causing a chemical reaction of a substrate.

(Technical concept 11) In the target molecule detection method according to technical concept 10, the enzyme, as the label connected to the second binding element, generates hydrogen ions by causing a hydrolysis reaction of the substrate.

The present disclosure is not limited to the embodiments described above. Various modifications can be made within the scope of the claims, and embodiments obtained by appropriately combining the technical means disclosed in different embodiments are also included within the technical scope of the present disclosure.

## Claims

1. A target molecule detection kit used to detect a target molecule (10), the target molecule detection kit comprising:
a first binding element (20) immobilized on a solid phase (30), the first binding element having reaction specificity to a first specific binding site of the target molecule; and
a second binding element (40) connected to a label (50) that generates a detection signal, the second binding element having reaction specificity to a second specific binding site of the target molecule that is different from the first specific binding site.

2. The target molecule detection kit according to claim 1, wherein
one of the first binding element and the second binding element is an aptamer and the other of the first binding element and the second binding element is an aptamer or an antibody.

3. The target molecule detection kit according to claim 1 or 2, wherein
the label connected to the second binding element is an enzyme that generates ions as the detection signal by causing a chemical reaction of a substrate.

4. The target molecule detection kit according to claim 3, wherein
the enzyme, as the label connected to the second binding element, generates hydrogen ions as the detection signal by causing a hydrolysis reaction of the substrate.

5. A target molecule detection method to detect a target molecule (10), the target molecule detection method comprising:
reacting the target molecule with a first binding element (20) immobilized on a solid phase, and a second binding element (40) connected to a label (50) that generates a detection signal, wherein
the first binding element has reaction specificity to a first specific binding site of the target molecule, and
the second binding element has reaction specificity to a second specific binding site of the target molecule that is different from the first specific binding site.

6. The target molecule detection method according to claim 5, further comprising:
extracting a complex (1) of the target molecule having bound to both the first binding element and the second binding element; and
detecting the target molecule using the label connected to the second binding element included in the extracted complex.

7. The target molecule detection method according to claim 6, wherein
the reacting of the target molecule with the first binding element and the second binding element includes:
reacting the target molecule with the first binding element, and then reacting the target molecule with the second binding element, or
reacting the target molecule with the second binding element, and then reacting the target molecule with the first binding element.

8. The target molecule detection method according to claim 6, wherein
the reacting of the target molecule with the first binding element and the second binding element includes causing a reaction between the target molecule and the first binding element and a reaction between the target molecule and the second binding element to occur at a same timing.

9. The target molecule detection method according to any one of claims 5 to 8, wherein
one of the first binding element and the second binding element is an aptamer and the other of the first binding element and the second binding element is an aptamer or an antibody.

10. The target molecule detection method according to any one of claims 5 to 9, wherein
the label connected to the second binding element is an enzyme that generates ions as the detection signal by causing a chemical reaction of a substrate.

11. The target molecule detection method according to claim 10, wherein
the enzyme, as the label connected to the second binding element, generates hydrogen ions as the detection signal by causing a hydrolysis reaction of the substrate.
